Europäisches Patentamt

European Patent Office

Office européen des brevets

(19)

(11) Publication number: **0 005 058**
**B1**

# EUROPEAN PATENT SPECIFICATION

(12)

(45) Date of publication of patent specification: **17.10.84**

(51) Int. Cl.³: **A 61 K 31/195** // **(A61K31/195, 31/195)**

(21) Application number: **79300650.3**

(22) Date of filing: **20.04.79**

(54) Composition for regulating dopamine or norepinephrine levels in neuronal synapses.

(30) Priority: **24.04.78 US 898740**

(43) Date of publication of application:
**31.10.79 Bulletin 79/22**

(45) Publication of the grant of the patent:
**17.10.84 Bulletin 84/42**

(84) Designated Contracting States:
**BE CH DE FR GB IT LU NL SE**

(56) References cited:

**CHEMICAL ABSTRACTS, vol. 83, no. 11, 15
September 1975, page 343, abstract no.
94224q, COLUMBUS OHIO (US).
Rote Liste 1976, 51009B, 51010B, 51029B,
51037B
Rote Liste 1974, 35001B, Unlisted Drugs 29
(1972), 113
Manufacturing Chemist and Aerosol News 36
(1960), 54
Chem. Abs 79 (1973), 87422s
Chem. Abs 85 (1976), 14103c**

(73) Proprietor: **MASSACHUSETTS INSTITUTE OF
TECHNOLOGY
77 Massachusetts Avenue
Cambridge, MA 02139 (US)**

(72) Inventor: **Wurtman, Richard J.
271 Woodward Street
Waban Massachusetts 02168 (US)**

(74) Representative: **Harvey, David Gareth et al
Graham Watt & Co. Riverhead
Sevenoaks Kent TN13 2BN (GB)**

Courier Press, Leamington Spa, England.

# 0 005 058

## Description

This invention is concerned with a composition in pharmaceutical unit dosage form for use in regulating (increasing or decreasing) the amount of dopamine or norepinephrine released into neuronal synapses in the brain.

It is known that the neurotransmitters dopamine and norepinephrine are derived from dihydroxyphenylalanine (DOPA). DOPA is, in turn, produced in neurons by the enzymatic hydroxylation of the amino acid tyrosine. This process is catalyzed by the enzyme tyrosine hydroxylase. The DOPA is decarboxylated to dopamine by the enzyme aromatic L-amino acid decarboxylase (AAAD) and norepinephrine is produced from dopamine in neurons that also contain the enzyme dopamine betahydroxylase. It is also known that within this reaction chain, the rate-limiting step is the conversion of tyrosine to DOPA. For this reason, DOPA has been administered to patients who suffer medical disability resulting from dopamine deficiency in diseases such as Parkinson's Disease. Unfortunately, DOPA, when administered, is taken up by cells throughout the body and converted to dopamine and this interferes with the normal metabolic processes in these other cells. In addition, DOPA interferes with the body's normal storage of the neurotransmitter serotonin, and lowers brain levels of the compound S-adenosylmethionine. It is believed that these effects contribute to such unwanted side-effects as the "On-Off Phenomenon" and, in some patients, psychotic symptoms. Other types of drugs that act by increasing dopamine and norepinephrine levels in synapses include the Monoamine Oxidase Inhibitors (which slow the destruction of these neutrotransmitters) and the tricyclic antidepressants; these compounds, which are used in treating diseases like depression; also relatively non-specific— producing many chemical effects besides increasing synaptic dopamine and norepinephrine levels— and thus have a range of unwanted side-effects such as the dangerous increases in blood pressure that occur when people receiving monoamine oxidase inhibitors eat certain foods.

Other diseases appear to be caused by the presence of excessive quantities of dopamine or norepinephrine within synapses including psychosis (too much dopamine), movement disorders like tardive dyskinesia and the Gilles de la Tourette Syndrome (too much dopamine), and hypertension and cardiac arrhythmias (too much norepinephrine released from sympathetic neurons). These diseases now usually are treated by drugs that block the interactions of dopamine or norepinephrine with their post-synaptic receptors, such as phenothiazines or butyrophenones. However, these agents all exhibit some non-specific actions as well, and thus cause side-effects.

Prior attempts to increase or decrease the levels of dopamine or norepinephrine by modifying neuronal tyrosine levels had been deemed unsuccessful because the total amounts of these compounds in brains and tissues were not noted to change. It was first observed in Wurtman et al (Science 185:183—184, July 12, 1974) that increases in brain DOPA concentrations could be obtained by increasing brain tyrosine concentrations, and that decreases in brain DOPA concentrations could be produced by giving rats treatments that decreased brain tyrosine. An example of a treatment that increased brain tyrosine was the administration of tyrosine itself; an example of a treatment that decreased brain tyrosine was the administration of one of the other neutral amino acids, e.g. leucine, that competes with plasma tyrosine for uptake into the brain. Prior to that disclosure, it had been believed that the rate-limiting enzyme, tyrosine hydroxylase, was so saturated with tyrosine, that increases or decreases in brain tyrosine levels would not affect tyrosine's conversion to DOPA. In neither the above Wurtman et al article nor a subsequent paper by Gibson and Wurtman (Biochem. Pharmacology, 26:1137—1142, June 1977) was it actually shown that such changes in DOPA accumulation were accompanied by changes in brain dopamine nor norepinephrine levels. Furthermore, in neither was it shown that changing brain tyrosine levels had any effect on the amounts of dopamine nor norepinephrine released into synapses.

It is an object of the present invention to provide a means for increasing or decreasing the amounts of dopamine and/or norepinephrine that actually are present within synapses. Such changes in synaptic transmitter levels need not be associated with changes in the total amounts of dopamine or norepinephrine present in the brain or other tissues.

It is another object of the invention to provide such a means which is biochemically specific and which lacks the undesirable side effects associated with administration of DOPA, the MAO inhibitors, the phenothiazines, and the other drugs described above. Such a means can by itself be therapeutic in various disease states. Alternatively, it can be used in combination with other drugs to amplify their therapeutic effects.

A therapeutic composition embodying the invention can be used for treating diseases associated with a deficiency or an excess of dopamine and/or norepinephrine in synapses. We have found that treatments that increase or decrease neuronal tyrosine levels can also cause corresponding increases or reductions in the amounts of dopamine or norepinephrine released into synapses. A composition in pharmaceutical unit dosage form containing tyrosine and at least one other amino acid selected from phenylalanine (tyrosine's precursor), tryptophan, leucine, isoleucine, valine, methionine, threonine and histidine, can be administered alone or in admixtures, with or without other drugs, in order to raise or lower brain tyrosine levels, and thereby to treat diseases associated with deficiency or excess of dopamine and/or norepinephrine in synapses. By varying the proportion of tryptophan, in the mixture,

2

the synthesis and synaptic release of serotonin, another brain neurotransmitter, can similarly be controlled. Increased intrasynaptic dopamine levels are obtained after tyrosine administration only when the dopamine-releasing neurons are active, i.e. are firing frequently. Increased synaptic norepinephrine levels can be obtained by giving tyrosine. Decreases in dopamine and norepinephrine released into synapses can be obtained by lowering brain tyrosine levels by administering amino acid compositions low in tyrosine levels. Decreases in serotonin release can similarly be obtained by lowering brain tryptophan levels. By regulating the proportion of tyrosine in a given mixture of amino acids, it can be caused to increase or decrease dopamine and/or norepinephrine release. Tryptophan's proportion in the amino acid mixture can be used to regulate serotonin's release into synapses while regulating dopamine and norepinephrine release as described herein.

In "The Lancet" for 6th February 1965 page 302 there is a contribution from Allan et al entitled "Treatment of Leukaemia by Amino Acid Imbalance" (see also "Manufacturing Chemist and Aerosol News" 36 (1960), page 54). Allan et al disclose the use of L-phenylalanine and L-tyrosine in equal proportions to treat childhook leukaemia. We disclaim compositions containing tyrosine and phenylalanine in which the phenylalanine is present in an amount equal to or greater than the amount of tyrosine.

In, for example, "Rote Liste" 1976, 51009B, 51010B, 51029B, 51037B; "Rote Liste" 1974, 35001B; and "Unlisted Drugs" 29 (1977) page 113, EAS, are disclosed certain amino-acid containing compositions for dietary purposes. In these compositions the phenylalanine is present in an amount greater than the tyrosine.

The invention provides a composition in pharmaceutical unit dosage form for use in therapy in regulating the amount of dopamine or norepinephrine released into synapses in the brain, characterised in that it contains tyrosine (or the ethyl ester of tyrosine) and at least one other amino acid selected from phenylalanine, tryptophan, leucine, isoleucine, valine, methionine, threonine and histidine, the composition being such that it Is effective to regulate blood plasma levels of tyrosine to form required amounts of dopamine and/or norephinephrine released into synapses in the brain, excluding compositions containing phenylalanine in which the phenylalanine is present in an amount equal to or greater than the amount of tyrosine.

The composition may be associated with a drug which induces an increase or decrease in dopaminergic or noradrenergic neurotransmission; this drug may be provided separately or in admixture with the composition.

The composition may also comprise an excipient.

The compositions are provided in unit dosage form.

The essentially dietary compositions of the "Rote Liste" references referred to above would not achieve the effect of the present invention.

Certain amino acids are essential in the diet for the maintenance of body tissues and for the growth of infants; in the healthy body, there is a natural balance of amino acids in the blood plasma. The dietary compositions are intended to achieve balance in the blood plasma, for example in the treatment of certain diseases, involving a deficiency of amino acids, where it is necessary to feed a patient parenterally (e.g. intravenously following an internal operation). The dietary compositions are necessarily tailored to achieve the natural balance of amino acids in the body and there is otherwise no special therapeutic effect intended or desired. Whatever the dosage of the dietary composition, the amino acids present compete for uptake into the brain, and there is for this reason no possibility of obtaining even by chance the therapeutic effect of the present invention.

In Allan et al also referred to above the L-phenylalanine and L-tyrosine in equal proportions were used in a daily dosage of 20—40 grams to treat the childhood leukaemia. Assuming 50 kilograms for the weight of a typical child, it follows that Allan discloses at least 200 mg/kg of tyrosine. This dosage of tyrosine by competition with other amino acids would starve the body of its normal amino acid diet preventing the production of body tissues. The effect observed by Allan was essentially toxic; creating an amino acid imbalance at cell level preventing the metabolism of the leukaemic cells. Even if Allan's composition did produce synaptic release of dopamine or norepinephrine, this would not be effective, since it would be swamped by the cytotoxic effect referred to.

Goldstein et al in a letter to "J. Pharm. Pharmac" 25 (1973), page 348 et seq and Sedvall et al in "Excerpta Med. Int. Congr." (Neuropsychopharmacology) 1975, Part 359, page 459 et seq. disclose the use of radio-labelled tyrosine but in the investigation of the effects of certain drugs, rather than for any therapeutic effect of the tyrosine itself. Neither reference discloses a composition containing another amino acid in addition to the tyrosine, and in Goldstein et al the radio-labelled tyrosine is used only in vitro.

There now follows a description to be read with reference to the accompanying drawings of embodiments of the invention. This description is given by way of example only, and not by way of limitation of the invention.

In the drawings:—

Figure 1 is a graph illustrating norepinephrine synthesis in the brains of rats; and

Figure 2 is a graph illustrating a correlation between homovanillic acid and brain tyrosine in rats.

Tyrosine and at least one other amino acid selected from phenylalanine, tryptophan, leucine, isoleucine, valine, methionine, threonine and histidine, is administered to a patient either alone or in combination with one or more other drugs thereby to increase or decrease the levels of dopamine and/or norepinephrine which are released into synapses. Serotonin release also can be controlled at the same time by varying the proportion of tryptophan present in the amino acid mixture. In order to increase dopamine release, it is necessary that the dopamine-releasing neurons in the patient's brain be relatively active, i.e. are firing frequently, such as is the case in patients with Parkinson's Disease. However, release of serotonin into synapses can be varied using amino acid mixtures whether or not the serotonin-releasing neurons are especially active. Similarly, decreases in dopamine or norephinephrine release can be produced by administering amino acid mixtures low in tyrosine so that the other amino acid(s) compete with tyrosine for uptake for the brain thereby decreasing brain tyrosine levels.

The composition of the amino acid mixture that is utilized depends upon the nature of the illness in the patient that is to be treated. When there is need to increase dopamine and/or norephinephrine release without increasing that of serotonin, tyrosine is administered, with at least one other of the specified amino acids but not including serotonin's precursor, tryptophan, in doses ranging between 5 mg/kg and 200 mg/kg. This therapy is useful, alone or as an adjunct to drug therapies, in treating Parkinson's Disease, certain types of depression, essential hypertension, or the low blood pressure that results from impaired peripheral sympathetic nervous function (orthostatic hypotension).

When there is need to sustain or increase brain serotonin levels while increasing dopamine or norephinephrine release, the compositions do contain tryptophan in addition to the tyrosine and (if present) phenylalanine and any of the other specified amino acids which may be present. This combination is especially useful in treating certain types of depression, or sleep disorders. It will be realized that the said other specified amino acids which these compositions can contain are the branched-chain amino acids (leucine, isoleucine, valine), as well as methionine, threonine and histidine.

When there is need to decrease synaptic dopamine levels, for example, in psychosis, or in such movement disorders as tardive dyskinesia or Gilles de la Tourette's Syndrome, i.e. diseases in which dopaminergic neurons are probably hyperactive, amino acid mixtures are administered which contain relatively little tyrosine or (if present) phenylalanine. Similar mixtures can be used to decrease synaptic norepinephrine, and to increase or decrease synaptic serotonin depending upon their tryptophan contents.

The ratios of the plasma concentrations of tyrosine, phenylalanine and tryptophan to the sum of the other neutral amino acids are normally 0.08—0.12, 0.07—0.12 and 0.06—0.14 respectively, depending on the composition of the diet. In some diseases, (e.g., cirrhosis of the liver leading to coma; diabetes; hyperinsulinism) such catabolic states as starvation, cachexia, disseminated cancer, or severe burns or trauma, these ratios are abnormal, causing changes in brain dopamine, norepinephrine and serotonin release. The particular compositions used in these situations are designed to restore the plasma ratios to normal. In the primarily neurologic or psychiatric diseases listed above, the goal of amino acid therapy is to raise or lower these ratios above or below their normal ranges, in order to increase or decrease the release of dopamine or norepinephrine (or serotonin) into synapses.

Compositions embodying the invention can be administered directly, or as constituents of foods. The route of administration can be oral or parenteral, e.g. intravenous.

Examples

The following Examples illustrate the activity of tyrosine in regulating the amount of dopamine or norepinephrine released into synapses in the brain but are not actual embodiments of the invention in that the tyrosine (or the ethyl ester of tyrosine) is used alone and not in conjunction with another amino acid.

Example I

This example illustrates that brain norepinephrine can be synthesized by increasing brain tyrosine levels.

This example shows that the rate at which 3-methoxy-4-hydroxy-phenylethyleneglycol-sulfate (MOPEG-SO$_4$), the major brain metabolite of norepinephrine, accumulates in rat brain also varies as a function of brain tyrosine levels. This shows that brain tyrosine levels affect not only the synthesis, but also the turnover and release of brain norephinephrine.

Male Sprague-Dawley rats (Charles River Breeding Laboratories, Wilmington, MA) weighing 150 g were housed in hanging cages (6—8 per cage), given ad libitum access to tap water and a 26% protein diet (Charles River Rat-Mouse-Hamster Maintenance Formula 24RF), and maintained under light (300 microwatts/cm$^2$; Vita-Lite, Duro-Test Corp., North Bergen, N.J.) between 8 AM and 8 PM daily. Rats used for diet experiments were fasted overnight and then allowed to consume the experimental diet starting at 10 AM. Diets of different compositions were prepared in agar gel (35 g/100 ml of water), as described by Gibson et al., Biochem. Pharmacol., 26, 1137—1142 (1977). All amino acids and drugs were injected intraperitoneally.

Norepinephrine synthesis and turnover in brain neurons were estimated by measuring the rate of

**0 005 058**

accumulation of MOPEG-SO$_4$ after probenecid administration or exposure to a cold environment. The MOPEG-SO$_4$ in brain homogenates was isolated using an anion exchange column (A-25 DEAE Sephadex; Pharmacia, Piscataway, N.J.); the method used was basically that of Meek and Neff, Br. J. Pharmacol., 45, 435—441 (1972), but modified to allow both tyrosine and MOPEG-SO$_4$ to be measured in the same sample. An aliquot of each homogenate (in 0.15 M ZnSO$_4$) was first assayed for tyrosine by the method of Waalkes and Udenfriend, J. Lab. Clin. Med., 50, 733—736 (1957). An equal volume of 0.15 M barium hydroxide was then added to the remaining homogenate, which was rehomogenized (Polytron, Brinkman Instruments, N.Y.), centrifuged and assayed for MOPEG-SO$_4$ by the method of Meek and Neff above. Recoveries of MOPEG-SO$_4$ and tyrosine from whole brain homogenates were 70—75% and 85—95% respectively.

Tyrosine (Grand Island Biological Co., Long Island, N.Y.) and probenecid (Sigma Chemical Co., St. Louis, MO), which are poorly soluble in water, were dissolved in dilute NaOH; the solutions were then buffered to pH 7.4 with hydrochloric acid and brought to a known volume with saline. This yielded a fine suspension that was suitable for injection.

In experiments on stress produced by exposure to cold, animals received the more soluble ethylester form of tyrosine (J. T. Baker, Phillipsburg, N.J.), instead of tyrosine itself, to raise brain tyrosine levels. Data were analyzed by one-way or two-way analysis of variance.

Probenecid treatment significantly raised the MOPEG-SO$_4$ level in brain from 123 ng/g in diluent-injected controls to 175 ng/g in probenecid-treated animals ($P < 0.001$) (Table I). Tyrosine administration alone had no effect on brain MOPEG-SO$_4$; however, pretreatment with this amino acid significantly enhanced the probenecid-induced rise in MOPEG-SO$_4$ (to 203 ng/g, as compared with 175 ng/kg in rats receiving probenecid alone ($P < 0.01$; Table I).

TABLE I

Accumulation of MOPEG-SO$_4$ after probenecid administration
and pretreatment with tyrosine

| Pretreatment | Brain tyrosine level ($\mu$g/g) | | Brain MOPEG-SO$_4$ level (ng/g) | |
|---|---|---|---|---|
| | Diluent | Probenecid | Diluent | Probenecid |
| Diluent | $13.9 \pm 0.5$ | $15.7 \pm 0.7$ | $123 \pm 6$ | $175 \pm 6$ |
| Tyrosine | $23.3 \pm 1.5$ | $24.7 \pm 1.3$ | $127 \pm 2$ | $203 \pm 8$ |

Note: In each of 3 experiments, groups of 4—6 rats were injected with either a dose of tyrosine (100 mg/kg, i.p.) known to accelerate brain dopa synthesis or its diluent and, 30 min. later, with probenecid (400 mg/kg, i.p.) or its diluent. Animals were killed 60 min. after the second injection, and their whole brains were analyzed for tyrosine and MOPEG-SO$_4$. Tyrosine administration significantly raised brain tyrosine levels ($P \pm 0.001$) whereas probenecid failed to modify brain tyrosine or its response to exogenous tyrosine. Probenecid significantly raised brain MOPEG-SO$_4$ ($P \pm 0.001$), and tyrosine pretreatment significantly enhanced this response ($P \pm 0.01$). Data were analyzed by two-way analysis of variance. Values are expressed as means $\pm$ SEM.

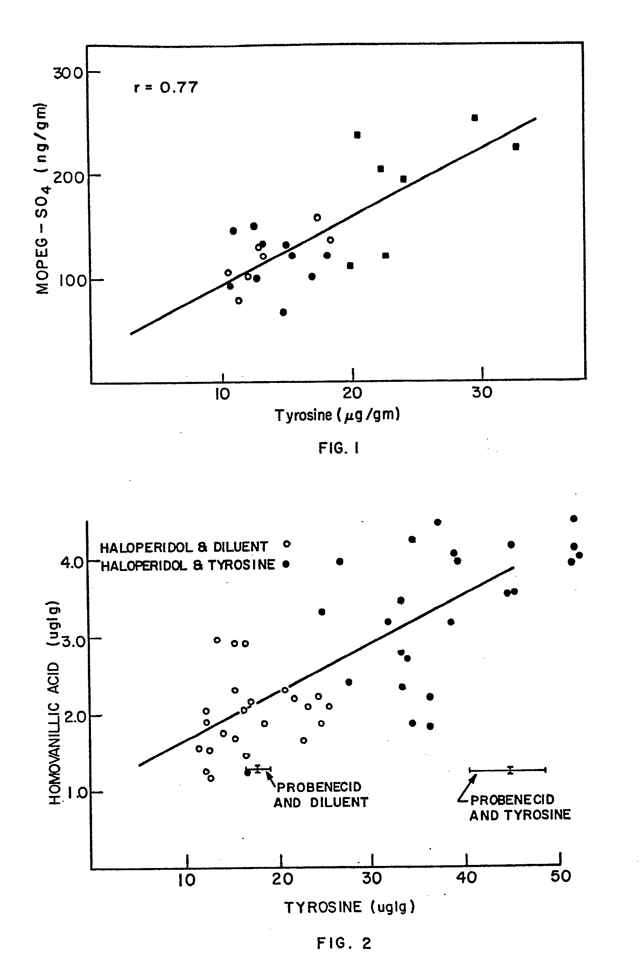

Placing the rats in a cold environment (4°C) increases norepinephrine turnover; this accelerates the formation of both norepinephrine itself and its metabolite, MOPEG-SO$_4$, in brain neurons. The rats were exposed to cold to determine whether treatments that changed brain tyrosine levels could influence the rate at which the brain accumulates MOPEG-SO$_4$ in rats exposed to cold stress and not given probenecid (Fig. 1).

Exposure to cold for 1 hr. increased brain MOPEG-SO$_4$ levels by about 40% (from 80 ng/g to 114 ng/g; $P < 0.01$). In animals treated with either of the amino acids or with saline, brain tyrosine levels paralleled, and were significantly correlated with, those of MOPEG-SO$_4$ ($r = 77$, $P < 0.05$; Fig. 1). Pretreatment with tyrosine raised brain tyrosine levels by about 80% (from 13.3 $\mu$g/g, in saline-injected animals, to 24.6 $\mu$g/g; $P < 0.01$) and those of MOPEG-SO$_4$ by 70% (from 114 ng/g to 193 ng/g; $P < 0.01$). Pretreatment with valine failed, in this study, to cause significant alterations in brain tyrosine or MOPEG-SO$_4$ levels (14.3 $\mu$g/g and 117 ng/g, respectively); however, brain tyrosine and MOPEG-SO$_4$ levels were also significantly correlated in these animals, as in other experimental groups (Fig. 1).

The relationship shown in Fig. 1 was obtained as follows: Groups of rats were injected intraperitoneally with valine (200 mg/kg), an amino acid that competes with tyrosine for uptake into the brain (8), or with tyrosine (125 mg/kg of the ethyl ester) or saline; 30 min. later they were placed in single cages in a cold (4°C) environment. After 1 hr., all animals were killed, and their whole brains were analyzed for tyrosine and MOPEG-SO$_4$. Control animals were injected with saline and left at room temperature (22°C), also in single cages, for 90 min. Each point represents the tyrosine and MOPEG-SO$_4$ levels present in a single brain. Data were pooled from several experiments. Brain tyrosine and MOPEG-

$SO_4$ levels in animals kept at room temperature were 14.6 $\mu g/g$ and 80 ng/g, respectively. In Fig. 1, the symbols are as follows: closed circles, animals pretreated with valine; open circles, animals pretreated with saline; closed squares, animals pretreated with tyrosine.

These data show that treatments that increased brain tyrosine levels can accelerate the accumulation of the norepinephrine metabolite MOPEG-$SO_4$ in the brains of rats pretreated with probenecid or exposed to a cold environment. They are compatible with the high Km of tyrosine hydroxylase for its substrate, relative to brain tyrosine concentrations. The enzyme is especially vulnerable to substrate limitation when it has been activated, inasmuch as activation selectively enhances its affinity for its cofactor.

MOPEG-$SO_4$ is the major metabolite of norepinephrine formed in rat brain and it is transported out of the brain by a probenecid-sensitive mechanism. After probenecid administration, MOPEG-$SO_4$ accumulates at a linear rate in rat brain for at least 60 min. Since brain norepinephrine levels remain constant during this interval, the rate of MOPEG-$SO_4$ accumulation provides a useful index of the rate of norepinephrine synthesis. This rate apparently is lower in unstressed, probenecid-treated rats than in animals placed in the cold, however, in both circumstances, it is dependent on brain tyrosine levels.

Example II

This example illustrates that brain dopamine release can be enhanced by increasing brain tyrosine levels.

To determine whether tyrosine levels also affect the synaptic release of catecholamine neurotransmitters, the effect of tyrosine administration on the accumulation of the dopamine metabolite, homovanillic acid (HVA) was examined in brains of animals pretreated either with probenecid (a compound that prevents the egress of organic acids from the cerebrospinal fluid, Spector and Lorenzo, 1974) or with haloperidol (a drug that blocks central dopaminergic receptors, Ungerstedt et al, 1969). It was found that increasing the brain tyrosine levels accelerates HVA accumulation in brains of haloperidol-treated animals, but *not* in animals given probenecid.

Male, 150 to 200 g Sprague-Dawley rats (Charles River Breeding Laboratories, Wilmington, MA) were exposed to light (Vita-Lite, Duro-Test Co., North Bergen, N.J.) between 9 AM and 9 PM daily and allowed access to Big Red Rat Chow (Charles River Breeding Laboratories) and water *ad libitum*. Drugs were injected intraperitoneally at a volume of 2 ml/kg body weight. Haloperidol was administered as a soluble preparation (Haldol, McNeil Laboratories, La Jolla, CA); tyrosine and probenecid (Sigma Chemical Co., St. Louis, MO) were dissolved in 1 N NaOH and adjusted to pH 10.0. Control animals received the appropriate diluents. Twenty minutes after an injection of tyrosine (100 mg/kg) or its diluent, the animals received haloperidol (2 mg/kg) or probenecid (200 mg/kg); 70 min. later they were killed by decapitation. Brains were quickly removed, and the striata were dissected out (Glowinski and Iverson, 1966), frozen on dry ice, and subsequently assayed for HVA. Tyrosine was assayed in homogenates of the remaining brain (Waalkes and Udenfriend, 1957). To affirm that tyrosine concentration in the homogenates of remaining brain are similar to those in striatum homogenates, brains were compared from groups of 6 animals treated, some of which had received tyrosine and/or haloperidol, and no statistical significance was detected.

Tyrosine hydroxylase activity was measured by a modification of the method of Waymire et al. (1971). Corpora striata were homogenized in 10 volumes of 0.05 M Tris-acetate buffer (pH 6.0), containing 0.2 per cent Triton X-100 (Harleco, Philadelphia, PA). The homogenates were centrifuged for 10 min at 10,000 g, and the supernatant fluids were collected for assay (Coyle, 1972). The assay medium contained, in a total volume of 110 $\mu l$: 50 $\mu l$ of a supernatant fluid; 65 nmoles of $DMPH_4$ (2-amino-4-hydroxy-6,7-dimethyl-5,6,7,8-tetrahydropteridine hydrochloride (synthetic cofactor obtained from Calbiochem, San Diego, CA); 29 nanomoles of pyridoxal phosphate; 4 nanomoles of 2-mercaptoethanol; 240 units of catalase; 0.01 millimoles phosphate buffer (pH 6.2); and 10 $\mu l$ of aromatic L-amino acid decarboxylase prepared from hog kidneys (Coyle, 1972). Samples were preincubated for 2 min. at 37°C.

The reaction was started by adding 10 $\mu l$ of L-1-[14]-C-tyrosine (specific activity, 0.90 $\mu Ci/nmole$) to a final concentration of 0.1 mM in the assay medium and then incubating the sample at 37°C for 30 min. The assay was stopped by the addition of 0.5 ml of 10 per cent trichloroacetic acid. The acidified medium was then shaken for 2 hours to recover the $^{14}CO_2$, which was trapped by folded filter paper strips placed in 0.2 ml of NCS tissue solubilizer (Amersham/Searle, Arlington Heights, IL). The strips were then transferred to scintillation vials containing 10 ml of Aquasol (New England Nuclear, Boston, MA), and their radioactivity was counted. Blanks utilized either boiled supernatant fluids or complete assay mixtures containing monoiodotyrosine (0.2 mM); both methods yielded similar results.

Tyrosine administration markedly potentiated (by 59 per cent; $P<0.001$) the accumulation of HVA in striata of haloperidol-treated rats (Fig. 2). It failed, however, to affect the HVA levels in animals given probenecid, even though brain tyrosine levels were elevated to an equivalent extent (Fig. 2). Among haloperidol-treated rats, striatal HVA and brain tyrosine concentrations were highly correlated ($r=0.70$; $P<0.01$) (Fig. 2); no such correlation was observed in probenecid-treated animals.

The failure of HVA accumulation (and thus of dopamine formation) to vary with brain tyrosine level in probenecid-treated rats may reflect the operation of a receptor-mediated feedback mechanism, which couples the activation of striatal dopamine receptors to a suppression of dopamine synthesis. It can be postulated that tyrosine administration initially enhances the synthesis and release of dopamine in striata of probenecid-treated animals, and that the consequent increase in the activation of dopamine receptors causes a feedback decrease in dopamine formation. The decrease in dopamine formation would lead to a fall in the production of HVA. This hypothetical feedback mechanism would fail to operate in haloperidol-treated animals; hence, tyrosine levels could continue to affect dopamine synthesis, even after dopamine release was accelerated.

To examine the possibility that the failure of tyrosine administration to accelerate striatal HVA accumulation in probenecid-treated rats resulted from a feedback change in the kinetic properties of tyrosine hydroxylase, we measured the enzyme's affinity for tyrosine and for its pterin cofactor, $DMPH_4$, on brain samples from each of our experimental groups. The prior administration of tyrosine failed to affect the Kms of tyrosine hydroxylase for tyrosine of $DMPH_4$ *in vitro* (Table I). As noted previously (Zivkovic et al, 1974; Zivkovic and Guidotti, 1974), haloperidol administration did decrease the enzyme's Km for $DMPH_4$ (Table II).

Fig. 2 of tyrosine administration on the accumulation of HVA in corpora striata of rats given haloperidol or probenecid. Rats received tyrosine (100 mg/kg) or its diluent followed in 20 min. by haloperidol (2 mg/kg) or probenecid (200 mg/kg); they were sacrificed 70 min. after the second injection. Data from individual animals receiving haloperidol are indicated by open circles; data from rats receiving haloperidol plus tyrosine are indicated by closed circles. Striatal HVA levels were highly correlated with brain tyrosine levels in all animals receiving haloperidol ($r=0.70$; $P<0.01$). In contrast, the striatal HVA levels of animals receiving probenecid alone ($n=17$) did not differ from those of rats receiving probenecid plus tyrosine ($n=11$). Brain tyrosine and striatal HVA concentrations in each group were (respectively); probenecid, $17.65 \pm 1.33$ and $1.30 \pm 0.10$ $\mu g/g$; probenecid plus tyrosine, $44.06 \pm 3.91$ and $1.31 \pm 0.11$ $\mu g/g$; haloperidol, $17.03 \pm 0.97$ and $2.00 \pm 0.10$ $\mu g/g$; and haloperidol plus tyrosine, $36.02 \pm 2.50$ and $3.19 \pm 0.20$ $\mu g/g$.

TABLE II

Effect of pretreatment with tyrosine or its diluent, plus
probenecid or haloperidol, on the Kms of striatal tyrosine hydroxylase
for tyrosine and $DMPH_4$

| Treatment | $K_m$ for tyrosine ($\mu M$) | $K_m$ for $DMPH_4$ (mM) |
|---|---|---|
| Probenecid | $53.9 \pm 2.2$ | $0.72 \pm 0.01$ |
| Probenecid plus tyrosine | $52.1 \pm 1.7$ | $0.67 \pm 0.06$ |
| Haloperidol | $48.1 \pm 1.9$ | $0.13 \pm 0.01$ |
| Haloperidol plus tyrosine | $48.4 \pm 1.1$ | $0.12 \pm 0.01$ |

Animals were treated as described for Fig. 2. Samples of striatum were assayed for tyrosine hydroxylase activity by using tyrosine concentrations of 0.125—1.0 mM and $DMPH_4$ concentrations of 0.1 to 0.5 mM. Haloperidol administration with or without tyrosine significantly reduced the $K_m$ of tyrosine hydroxylase for $DMPH_4$, as compared to that observed in probenecid-treated animals ($P<0.001$, Student's t-test).

These data provide further support for the hypothesis that tyrosine hydroxylase may not always be saturated with its amino acid substrate *in vivo*—i.e., in animals whose brains are synthesizing larger-than-normal quantities of dopamine as a consequence of dopamine-receptor blockade (by haloperidol). Moreover, they show that increasing the saturation of the enzyme (by administering tyrosine) can enhance not only the formation of dopa and of the catecholamine neurotransmitter, dopamine, but also the release of this transmitter. Control by tyrosine of dopamine synthesis and release can be expected to operate whenever dopaminergic neurons are firing frequently, e.g., in Parkinson's Disease; after haloperidol.

**Claims**

1. A composition in pharmaceutical unit dosage form for use in therapy in regulating the amount of dopamine or norepinephrine released into synapses in the brain, characterised in that it contains tyrosine (or the ethyl ester of tyrosine) and at least one other amino acid selected from phenylalanine, tryptophan, leucine, isoleucine, valine, methionine, threonine and histidine, the composition being such that it is effective to regulate blood plasma levels of tyrosine to form required amounts of dopamine

7

**0 005 058**

and/or norepinephrine released into synapses in the brain, excluding compositions containing phenylalanine in which the phenylalanine is present in an amount equal to or greater than the amount of tyrosine.

2. A composition according to claim 1, wherein tryptophan is present in an amount to increase synaptic release of serotonin.

3. A composition according to claim 1, wherein tryptophan is present in an amount to decrease synaptic release of serotonin.

4. A composition according to claim 1, for use in the treatment of a disease that causes dopamine-releasing neurons to be active.

5. A composition according to claim 1, characterised by association with a drug which induces an increase or decrease in dopaminergic neurotransmission.

6. A composition according to claim 1, characterised by association with a drug which induces an increase in noradrenergic neurotransmission.

7. A composition according to claim 1, characterised by association with a drug which induces a decrease in noradrenergic neurotransmission.

8. A composition according to any one of claims 1, 2 and 6 for use in the treatment of depression, said composition being effective to release required amounts of norepinephrine into synapses in the brain.

9. A composition according to any one of claims 1 to 8, also comprising an excipient.

**Patentansprüche**

1. Zusammensetzung in Form pharmazeutischer Dosiseinheiten zur therapeutischen Verwendung bei der Regulierung der Mengen an Dopamin oder Norepinephrin, die in Gehirnsynapsen gelangen, dadurch gekennzeichnet, dass sie Tyrosin oder dessen Ethylester sowie mindestens eine andere Aminosäure enthält, ausgewählt aus Phenylalanin, Tryptophan, Leucin, Isoleucin, Valin, Methionin, Threonin und Histidin, und dass die Zusammensetzung derart beschaffen ist, dass sie die Regulierung des Tyrosinspiegels im Blutplasma bewirkt, indem die zur Ausschüttung von Dopamin und/oder Norepinephrin in Gehirnsynapsen erforderlichen Mengen dieser Stoffe gebildet werden, und wobei Zusammensetzungen ausgeschlossen sind, die gleich viel oder mehr Phenylalanin als Tyrosin enthalten.

2. Zusammensetzung nach Anspruch 1, welche Tryptophan in einer Menge enthält, die die synaptische Ausschüttung von Serotonin steigert.

3. Zusammensetzung nach Anspruch 1, welche Tryptophan in einer Menge enthält, die die synaptische Ausschüttung von Serotonin vermindert.

4. Zusammensetzung nach Anspruch 1 zur Verwendung in einer Krankheit, bei welcher dopaminfreisetzende Neuronen aktiviert werden.

5. Zusammensetzung nach Anspruch 1, dadurch gekennzeichnet, dass sie zusammen mit einem Medikament vorliegt, welches eine Steigerung oder eine Verminderung dopaminergischer Neurotransmission induziert.

6. Zusammensetzung nach Anspruch 1, dadurch gekennzeichnet, dass sie zusammen mit einem Medikament vorliegt, welches eine Steigerung der noradrenergischen Neurotransmission induziert.

7. Zusammensetzung nach Anspruch 1, dadurch gekennzeichnet, dass sie zusammen mit einem Medikament vorliegt, welches eine Verminderung der noradrenergischen Neurotransmission induziert.

8. Zusammensetzung nach einem der Ansprüche 1, 2 und 6 zur Verwendung bei der Behandlung von Depressionen, wobei die Zusammensetzung die Ausschüttung erforderlicher Mengen von Norepinephrin in Gehirnsynapsen bewirkt.

9. Zusammensetzung nach einem der Ansprüche 1 bis 8, welche weiterhin einen Exzipienten enthält.

**Revendications**

1. Composition sous forme d'unités de dosage pharmaceutique pour l'utilisation dans une thérapie réglant la quantité de dopamine ou de norépinephrine libérée dans des synapses cérébrales, caractérisée en ce qu'elle contient de la tyrosine ou son ester éthylique et au moins un autre acide aminé choisi parmis la phénylalanine, le tryptophane, la leucine, l'isoleucine, la valine, la méthionine, la thréonine et l'histidine, la composition étant telle qu'elle est efficace pour régler le taux de tyrosine dans le plasma sanguin pour former les quantités nécessaires de dopamine et/ou de norépinephrine libérées dans des synapses cérébrales, à l'exclusion des compositions contenant de la phénylalanine dans lesquelles la phénylalanine est présente en quantité égale ou supérieure à celle de la tyrosine.

2. Composition selon la revendication 1, dans laquelle le tryptophane est présent en une quantité apte à augmenter la libération synaptique de sérotonine.

3. Composition selon la revendication 1, dans laquelle le tryptophane est présent en une quantité apte à diminuer la libération synaptique de sérotonine.

4. Composition selon la revendication 1 pour son utilisation dans le traitement de maladies qui causent l'activation de neurons libérant de la dopamine.

8

5. Composition selon la revendication 1, caractérisée par l'association avec un médicament qui déclenche une augmentation ou une diminution de la neurotransmission dopaminergique.

6. Composition selon la revendication 1, caractérisée par l'association avec un médicament qui déclenche une augmentation de la neurotransmission noradrénergique.

7. Composition selon la revendication 1, caractérisée par l'association avec un médicament qui déclenche une diminution de la neurotransmission nroadrénergique.

8. Composition selon l'une quelconque des revendications 1, 2 et 6 pour l'utilisation dans le traitement de dépressions, la dite composition étant efficace pour libérer des quantités requises de norépinéphrine dans des synapses cérébrales.

9. Compositions selon l'une quelconque des revendications 1 à 8, comprenant également un excipient.

FIG. I

FIG. 2

1